# EUROPÄISCHE PATENTSCHRIFT

(11) **EP 3 355 967 B1**
(45) Veröffentlichungstag und Bekanntmachung des Hinweises auf die Patenterteilung: **04.12.2019**
(21) Anmeldenummer: 16787324.9
(22) Anmeldetag: 26.09.2016
(51) Int. Cl.: A61M 5/20, A61M 5/315, A61M 5/31

(54) **INJEKTIONSGERÄT**
INJECTION APPARATUS
DISPOSITIF D'INJECTION

(30) Priorität: 30.09.2015 DE 202015006841 U
(43) Veröffentlichungstag der Anmeldung: 08.08.2018
(73) Patentinhaber: Haselmeier AG, 9001 St. Gallen (CH)
(72) Erfinder: KEITEL, Joachim, 73728 Esslingen (DE)
(74) Vertreter: Reinhardt, Annette
(86) Internationale Anmeldenummer: PCT/EP2016/001600
(87) Internationale Veröffentlichungsnummer: WO 2017/054917

(56) Entgegenhaltungen:
- DE-U1-202012 001 411

## Beschreibung

Die Erfindung betrifft ein Injektionsgerät der im Oberbegriff des Anspruchs 1 angegebenen Gattung.

Aus der DE 20 2012 001 411 U1 ist ein Injektionsgerät der gattungsgemäßen Art bekannt, bei dem zum Einstellen einer auszupressenden Menge an Injektionsflüssigkeit ein Bedienelement gegenüber dem Gehäuse gedreht wird. Dabei dreht sich ein im Gehäuse drehbar gelagertes Dosierorgan mit. Zwischen dem Bedienelement und einer drehfest im Gehäuse gelagerten Injektionshülse wirkt eine Rasteinrichtung. Eine zweite Rasteinrichtung wirkt zwischen dem Gehäuse und einem drehbar im Gehäuse gelagerten Dosierorgan. Beim Einstellen einer auszupressenden Menge von Injektionsflüssigkeit sind beide Rasteinrichtungen wirksam, während beim Auspressen von Injektionsflüssigkeit nur die zweite Rasteinrichtung zwischen Gehäuse und Dosierorgan wirksam ist. Die zweite Rasteinrichtung ist vergleichsweise schwach ausgelegt, da der Bediener beim Einstellen einer auszupressenden Menge an Injektionsflüssigkeit beide Rasteinrichtungen zum Verdrehen des Bedienelementes überwinden muss.

Der Erfindung liegt die Aufgabe zugrunde, ein Injektionsgerät der gattungsgemäßen Art zu schaffen, das einen erhöhten Bedienkomfort besitzt.

Diese Aufgabe wird durch ein Injektionsgerät mit den Merkmalen des Anspruchs 1 gelöst.

Es ist vorgesehen, dass in einer ersten Position des Bedienelementes beim Einstellen einer auszupressenden Menge an Injektionsflüssigkeit eine erste Rasteinrichtung wirkt. Die erste Rasteinrichtung wirkt dabei nur in der ersten Position des Bedienelementes.

Außerdem ist eine zweite Rasteinrichtung zwischen dem Bedienelement und dem Dosierorgan vorgesehen, die nur in einer zweiten Position des Bedienelements beim Auspressen einer auszupressenden Menge an Injektionsflüssigkeit wirksam ist. Dadurch, dass in jeder Position des Bedienelements nur eine der beiden Rasteinrichtungen wirksam ist, können die Rasteinrichtungen unabhängig voneinander ausgelegt werden. Die erste Rasteinrichtung kann so ausgelegt werden, dass der Bediener eine ausreichende haptische und akustische Rückmeldung beim Einstellen einer auszupressenden Menge an Injektionsflüssigkeit erhält. Es kann vorteilhaft sein, dass die erste Rasteinrichtung so ausgelegt ist, dass der Bediener eine einmal eingestellte Menge an Injektionsflüssigkeit durch Drehen des Bedienelements in die Gegenrichtung auch wieder verringern kann. Die zweite Rasteinrichtung kann auf die beim Auspressen einer eingestellten Menge an Injektionsflüssigkeit gewünschte akustische Rückmeldung ausgelegt werden. Dadurch, dass die zweite Rasteinrichtung beim Einstellen einer auszupressenden Menge an Injektionsflüssigkeit nicht aktiv ist, ist es möglich, die zweite Rasteinrichtung vergleichsweise stark auszulegen, so dass sich eine deutliche akustische Rückmeldung beim Auspressen von Injektionsflüssigkeit ergibt. Vorteilhaft sind die beiden Rasteinrichtungen dabei vollständig getrennt voneinander ausgebildet. Dadurch können die Rasteinrichtungen unabhängig voneinander ausgelegt werden.

Eine Rasteinrichtung ist dabei wirksam, wenn die Rasteinrichtung beim Einstellen einer auszupressenden Menge an Injektionsflüssigkeit bzw. beim Auspressen einer auszupressenden Menge an Injektionsflüssigkeit, also wenn sich die Bauteile in der vorgesehenen Weise relativ zueinander bewegen, eine akustische und/oder haptische Rückmeldung gibt, also vorteilhaft Klicklaute erzeugt und die Bauelemente relativ zueinander in vorgegebene Rastpositionen stellt. Eine Rasteinrichtung ist nicht wirksam, wenn sich die Bauteile, zwischen denen die Rasteinrichtung wirkt, nicht in Rastrichtung zueinander bewegen, so dass keine Rastlaute erzeugt und die Elemente in Rastschritte verstellt werden.

Vorteilhaft dreht sich das Dosierorgan beim Einstellen einer auszupressenden Menge an Injektionsflüssigkeit in einer ersten Drehrichtung gegenüber dem Gehäuse und beim Auspressen einer eingestellten Menge an Injektionsflüssigkeit in einer der ersten Drehrichtung entgegen gerichteten zweiten Drehrichtung. Vorteilhaft ist die zweite Rasteinrichtung so ausgelegt, dass sie eine Drehung des Dosierorgans gegenüber dem Bedienelement in der zweiten Drehrichtung erlaubt und eine Drehung des Dosierorgans gegenüber dem Bedienelement in der ersten Drehrichtung sperrt. Dadurch ist eine Drehung des Dosierorgans in der ersten Drehrichtung nicht möglich, wenn sich das Bedienelement in der zweiten Position befindet. Vorteilhaft ist das Injektionsgerät ein automatisch wirkendes Injektionsgerät, bei dem nach dem Einstellen einer auszupressenden Menge an Injektionsflüssigkeit ein Auslöseelement betätigt wird und die Injektion anschließend automatisch abläuft. Hierzu ist vorteilhaft vorgesehen, dass das Injektionsgerät eine Feder besitzt, die beim Einstellen einer auszupressenden Menge an Injektionsflüssigkeit gespannt wird und die bei gelöster erster Kupplung das Auspressen von Injektionsflüssigkeit bewirkt.

Eine vorteilhafte, einfache Gestaltung ergibt sich, wenn das Bedienelement mindestens einen Raststeg besitzt, wobei der mindestens eine Raststeg in der ersten Position des Bedienelements einen Teil der ersten Kupplung bildet und in der zweiten Position des Bedienelements einen Teil der zweiten Rasteinrichtung bildet. Der Raststeg besitzt somit eine Doppelfunktion. Dadurch ergibt sich ein einfacher und kompakter Aufbau.

Vorteilhaft besitzt das Injektionsgerät einen Mitnehmer, der drehfest mit dem Dosierorgan verbunden ist. Die zweite Rasteinrichtung ist bevorzugt an dem Bedienelement und dem Mitnehmer ausgebildet. Vorteilhaft ist auch die erste Kupplung an dem Bedienelement und dem Mitnehmer ausgebildet.

Ein bevorzugter Aufbau des Injektionsgerätes ergibt sich, wenn das Injektionsgerät einen Dosierkolben besitzt, der drehfest mit dem Bedienelement verbunden ist und der über eine erste Gewindeverbindung mit dem Dosierorgan verbunden ist. Beim Einstellen einer auszupressenden Menge an Injektionsflüssigkeit wird der Dosierkolben mit dem Bedienelement gedreht. Das Dosierorgan dreht sich mit dem Bedienelement, so dass die erste Gewindeverbindung beim Einstellen einer auszupressenden Menge an Injektionsflüssigkeit wirkungslos ist. Beim Auspressen einer auszupressenden Menge an Injektionsflüssigkeit ist das Bedienelement und mit dem Bedienelement auch der Dosierkolben drehfest im Gehäuse geführt. Die erste Gewindeverbindung bewirkt deshalb beim Auspressen einer auszupressenden Menge an Injektionsflüssigkeit eine axiale Bewegung des Dosierkolbens, die zum Auspressen von Injektionsflüssigkeit führt.

Das Injektionsgerät besitzt vorteilhaft eine Injektionshülse, die drehfest und in Richtung der Längsmittelachse verschiebbar gegenüber dem Gehäuse gehalten ist und die über eine zweite Gewindeverbindung mit dem Dosierorgan verbunden ist. Wenn das Bedienelement und damit das Dosierorgan gedreht werden, um die auszupressende Menge an Injektionsflüssigkeit einzustellen, bewegt sich die Injektionshülse aufgrund der zweiten Gewindeverbindung und der drehfesten Verbindung mit dem Gehäuse gegenüber dem Gehäuse in distale Richtung. Während der Injektion wird die Injektionshülse in proximale Richtung geschoben und versetzt damit das Dosierorgan in Drehung. Die Injektionshülse kann deshalb vorteilhaft auch zum Spannen einer Feder bei der Vorwahl der Dosis genutzt werden, welche Feder dann zum Auspressen der auszupressenden Menge an Injektionsflüssigkeit dient. Die Injektionshülse dient vorteilhaft weiterhin dazu, für den Bediener einen Bereich am Außenumfang des Dosierorgans, auf dem eine Skala abgebildet ist, sichtbar zu machen. Die zweite Gewindeverbindung ist so ausgelegt, dass die Injektionshülse jeweils die eingestellte Menge an Injektionsflüssigkeit anzeigt.

Ein Ausführungsbeispiel der Erfindung wird im Folgenden anhand der Zeichnung erläutert. Es zeigen:
- Fig. 1: eine Seitenansicht eines Injektionsgeräts in Nullstellung,
- Fig. 2: einen Schnitt entlang der Linie II-II in Fig. 1,
- Fig. 3: den Ausschnitt III aus Fig. 2 in vergrößerter Darstellung,
- Fig. 4: einen Schnitt entlang der Linie IV-IV in Fig. 3,
- Fig. 5: einen Schnitt entlang der Linie V-V in Fig. 3,
- Fig. 6: eine Seitenansicht des Injektionsgeräts aus Fig. 1 nach dem Auspressen einer eingestellten Menge an Injektionsflüssigkeit,
- Fig. 7: einen Schnitt entlang der Linie VII-VII in Fig. 6,
- Fig. 8: den Ausschnitt VIII aus Fig. 7 in vergrößerter Darstellung,
- Fig. 9: einen Schnitt entlang der Linie IX-IX in Fig. 8,
- Fig. 10: einen Schnitt entlang der Linie X-X in Fig. 8,
- Fig. 11: eine Seitenansicht des oberen Gehäuseteils des Injektionsgeräts,
- Fig. 12: einen Schnitt entlang der Linie XII-XII in Fig. 11,
- Fig. 13: einen Schnitt entlang der Linie XIII-XIII in Fig. 12,
- Fig. 14: eine perspektivische Darstellung des Bedienelements des Inj ektionsgeräts,
- Fig. 15: eine Seitenansicht des Bedienelements,
- Fig. 16: einen Schnitt entlang der Linie XVI-XVI in Fig. 15,
- Fig. 17: einen Schnitt entlang der Linie XVII-XVII in Fig. 15,
- Fig. 18 und 19: perspektivische Darstellungen eines Mitnehmers des Injektionsgeräts,
- Fig. 20: eine Seitenansicht des Mitnehmers aus den Fig. 18 und 19,
- Fig. 21: einen Schnitt entlang der Linie XXI-XXI in Fig. 20,
- Fig. 22: einen Schnitt entlang der Linie XXII-XXII in Fig. 20,
- Fig. 23: einen Schnitt entlang der Linie XXIII-XXIII in Fig. 20,
- Fig. 24: eine Seitenansicht des Dosierorgans des Injektionsgeräts,
- Fig. 25: einen Schnitt entlang der Linie XXV-XXV in Fig. 24,
- Fig. 26: einen Schnitt entlang der Linie XXVI-XXVI in Fig. 24,
- Fig. 27: eine Seitenansicht der Injektionshülse des Injektionsgeräts,
- Fig. 28: einen Schnitt entlang der Linie XXVIII-XXVIII in Fig. 27,
- Fig. 29: einen Schnitt entlang der Linie XXIX-XXIX in Fig. 27.

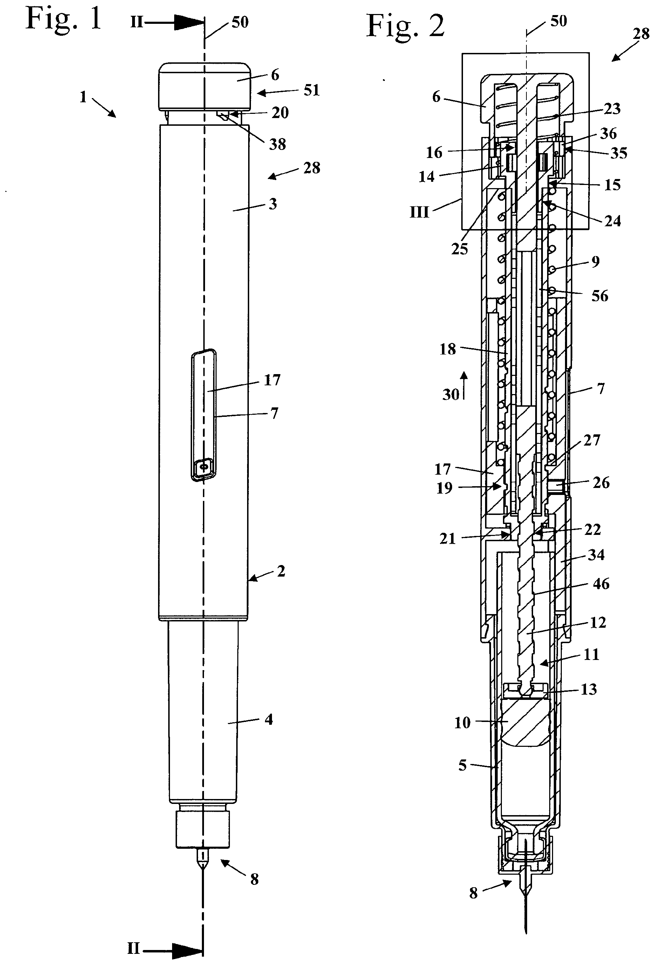

Fig. 1 zeigt ein Injektionsgerät 1 in Seitenansicht. Das Injektionsgerät 1 ist ein mechanisches Injektionsgerät, bei dem das Auspressen einer Dosis von Injektionsflüssigkeit nach dem Betätigen eines Auslöseelements automatisch erfolgt. Das Injektionsgerät 1 besitzt ein Gehäuse 2, das ein oberes Gehäuseteil 3 und einen am oberen Gehäuseteil 3 festgelegten Halter 4 umfasst. Der Halter 4 ist an einer proximalen Seite des oberen Gehäuseteils 3 angeordnet. An der proximalen Seite des Halters 4 ist eine Injektionsnadel 8 festgelegt. Mit "proximal" wird dabei die Seite des Injektionsgeräts 1 bezeichnet, die bei einer Injektion der Einstichstelle zugewandt liegt, und mit "distal" die Seite, die der Einstichstelle abgewandt liegt. Das distale Ende des Injektionsgeräts 1 ist das einer am Injektionsgerät 1 gehaltenen Injektionsnadel 8 abgewandt liegende Ende. Die proximale Richtung bezeichnet die Injektionsrichtung, also die Richtung zur Injektionsnadel 8 bzw. die Richtung, in der die Injektionsflüssigkeit aus einem Behälter ausgepresst wird. Die distale Richtung bezeichnet die entgegengesetzte Richtung, also von einer am Gehäuse 2 angeordneten Injektionsnadel 8 weg.

An der distalen Seite des Injektionsgeräts 1 ist ein Bedienelement 6 angeordnet. Das Bedienelement 6 ist über eine Kupplung 20 mit dem oberen Gehäuseteil 3 drehfest verbindbar. Wie Fig. 1 zeigt, umfasst die Kupplung 20 Stege 38, von denen in Fig. 1 einer sichtbar ist, und die in eine entsprechende Kontur am oberen Gehäuseteil 3 ragen, um das Bedienelement 6 mit dem oberen Gehäuseteil 3 drehfest zu verbinden. Das obere Gehäuseteil 3 besitzt ein Sichtfenster 7, das vorteilhaft aus durchsichtigem Material besteht, so dass durch das Sichtfenster 7 eine im oberen Gehäuseteil 3 angeordnete Injektionshülse 17 sichtbar ist. Das Injektionsgerät 1 besitzt eine Längsmittelachse 50, die in Längsrichtung des Gehäuses 2 des Injektionsgeräts 1 verläuft.

Die Fig. 1 und 2 zeigen das Injektionsgerät 1 in einer Nullstellung 28, in der keine Dosis von Injektionsflüssigkeit eingestellt ist. Das Bedienelement 6 befindet sich in einer ersten, distalen Position 51, die die distale Endlage des Bedienelements 6 ist. Wie Fig. 2 zeigt, ist im Halter 4 ein Behälter 5 mit Injektionsflüssigkeit angeordnet. Im Behälter 5 ist ein Stopfen 10 angeordnet, an dem eine Kolbenscheibe 13 eines Dosierkolbens 11 anliegt. Der Dosierkolben 11 umfasst außerdem eine Kolbenstange 12, die ein Außengewinde 46 trägt.

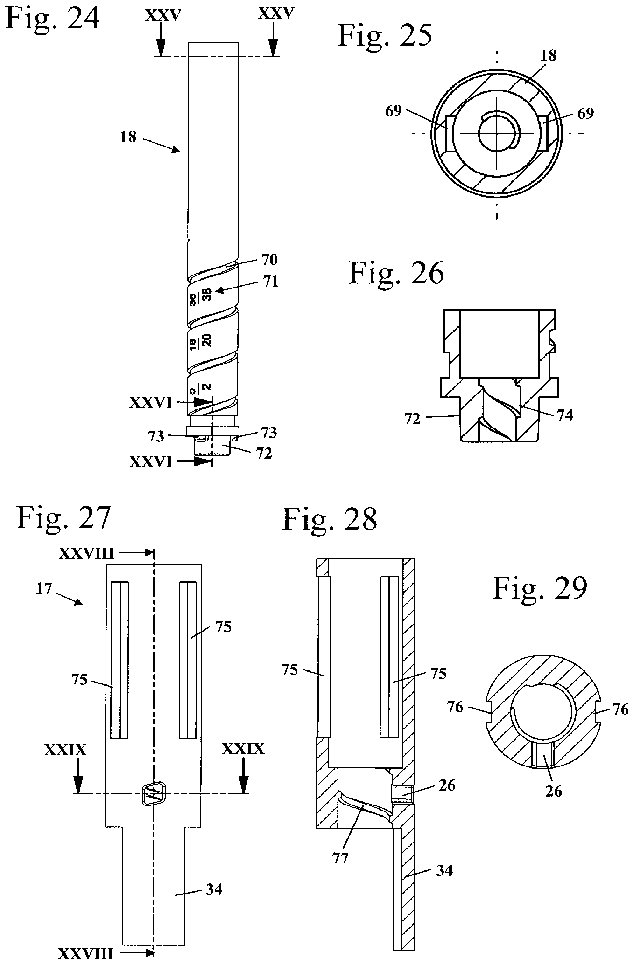

Die Außenseite der Injektionshülse 17 ist durch das Sichtfenster 7 des oberen Gehäuseteils 3 sichtbar. Die Injektionshülse 17 besitzt eine Öffnung 26, durch die der Außenumfang eines radial innerhalb der Injektionshülse 17 angeordneten Dosierorgans 18 sichtbar ist. Das Dosierorganl8, das auch als Skalenrohr bezeichnet werden kann, trägt an seinem Außenumfang eine in Fig. 24 gezeigte Skala 71, die durch das Sichtfenster 7 und die Öffnung 26 für den Bediener sichtbar ist und die eingestellte Menge von auszupressender Injektionsflüssigkeit anzeigt.

Das Dosierorgan 18 ist an einem Drehlager 21 drehbar im oberen Gehäuseteil 3 gelagert und axial unverschiebbar im oberen Gehäuseteil 3 gehalten. Das Dosierorgan 18 ist über eine erste Gewindeverbindung 22 mit dem Außengewinde 46 der Kolbenstange 12 verbunden. Die Injektionshülse 17 ist im oberen Gehäuseteil 3 in Richtung der Längsmittelsachse 50 verschiebbar und gegenüber dem oberen Gehäuseteil 3 drehfest gehalten. Die Injektionshülse 17 ist dabei in jeder Stellung des Injektionsgeräts 1 vollständig innerhalb des Gehäuses 2, nämlich innerhalb des oberen Gehäuseteils 3, angeordnet. Das Dosierorgan 18 und die Injektionshülse 17 sind über eine zweite Gewindeverbindung 19 miteinander verbunden.

Im oberen Gehäuseteil 3 ist ein Mitnehmer 14 drehbar gelagert. Der Mitnehmer 14 ist an einem im oberen Gehäuseteil 3 ausgebildeten Drehlager 15 drehbar gelagert, das durch einen Rand des oberen Gehäuseteils 3 gebildet wird. Der Mitnehmer 14 ist über eine drehfeste Verbindung 24 drehfest mit dem Dosierorgan 18 verbunden. Die drehfeste Verbindung 24 kann beispielsweise eine Pressverbindung oder eine formschlüssige Verbindung sein.

Wie Fig. 2 auch zeigt, ist im oberen Gehäuseteil 3 eine Feder 9 angeordnet, die als Druckfeder, nämlich als Schraubendruckfeder ausgebildet ist. Die Feder 9 stützt sich mit einem ersten Ende an einem Anlagerand 27 der Injektionshülse 17 ab und mit ihrem zweiten Ende an einem Anlagerand 25 des oberen Gehäuseteils 3. Am Anlagerand 25 ist auch das Drehlager 15 für den Mitnehmer 14 ausgebildet. Die Feder 9 ist radial außerhalb des Dosierorgans 18 angeordnet und ragt in der in Fig. 2 gezeigten Nullstellung 28 mit ihrem proximalen Bereich in einen zwischen Injektionshülse 17 und Dosierorgan 18 gebildeten Ringraum.

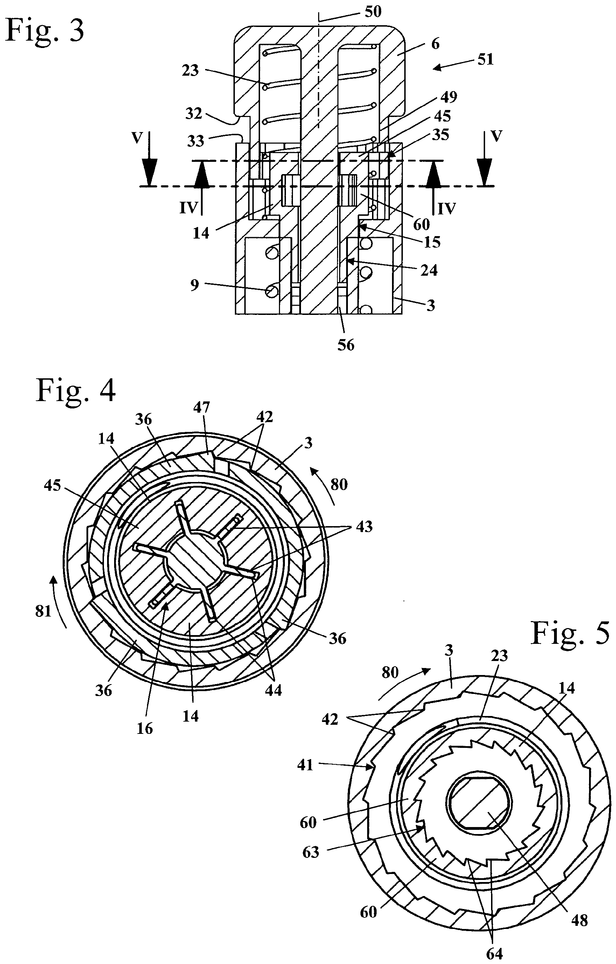

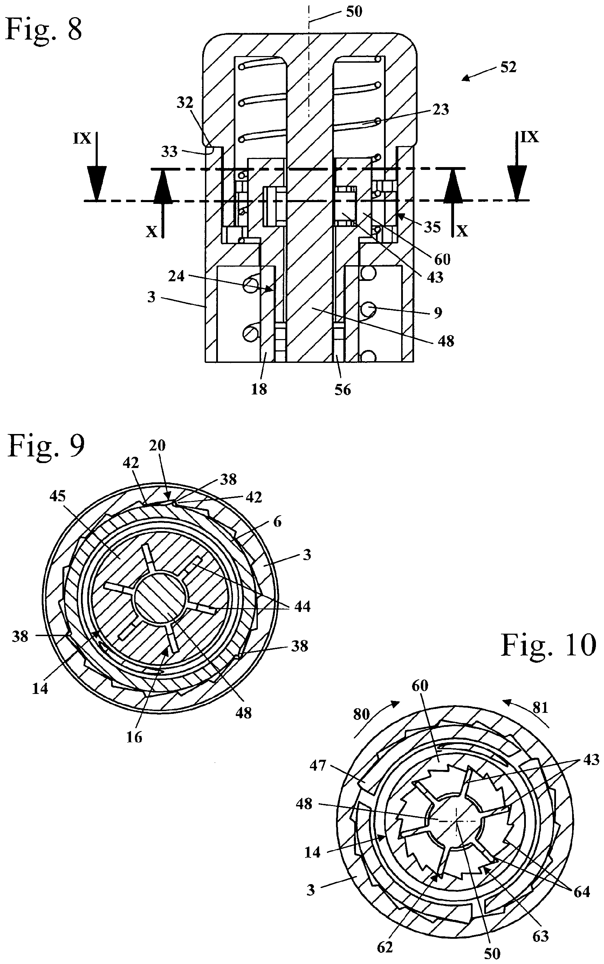

Das Bedienelement 6 ist über ein Verbindungselement 56, das im Ausführungsbeispiel als Hülse ausgebildet ist, drehfest mit der Kolbenstange 12 verbunden. Das Bedienelement 6 stützt sich über eine Feder 23, die als Schraubendruckfeder ausgebildet ist, gegenüber dem oberen Gehäuseteil 3 ab. Die Feder 23, die das Bedienelement 6 in distale Richtung drückt, hat auf die Geschwindigkeit der Injektion keinen Einfluss. Die Feder 23 ist lediglich so ausgelegt, dass der Bediener das Bedienelement 6 mit angenehmer Kraft aus der in den Figuren 1 und 2 gezeigten Nullstellung 28 in proximale Richtung bewegen kann. Wie Fig. 3 zeigt, ist am Bedienelement 6 ein Absatz 32 ausgebildet. Das obere Gehäuseteil 3 besitzt an seiner distalen Stirnseite einen Rand 33. Bei in proximale Richtung gedrücktem Bedienelement 6 wirkt der Absatz 32 mit einem Rand 33 des oberen Gehäuseteils 3 zusammen und bildet mit diesem einen Anschlag, der eine proximale Position 52 (Fig. 8) des Bedienelements 6 festlegt. Zur Festlegung der distalen Position 51 des Bedienelements 6 ist vorteilhaft ein weiterer Anschlag vorgesehen, der beispielsweise am Mitnehmer 14 ausgebildet sein kann.

Zwischen dem Bedienelement 6 und dem oberen Gehäuseteil 3 wirkt eine Rasteinrichtung 35 (Fig. 2), die mehrere Rastarme 36 umfasst. In Fig. 2 ist einer der Rastarme 36 sichtbar. Das Injektionsgerät 1 besitzt außerdem eine Kupplung 16, die in der in den Figuren 1 und 2 gezeigten Nullstellung 28 das Bedienelement 6 und den Mitnehmer 14 drehfest miteinander koppelt.

Bei unbetätigtem Bedienelement 6 drückt die Feder 23 das Bedienelement 6 in seine erste, distale Position 51. In dieser Position des Bedienelements 6 ist die Kupplung 20 geöffnet und das Bedienelement 6 gegenüber dem Gehäuse 2 drehbar. Zum Einstellen einer auszupressenden Menge an Injektionsflüssigkeit dreht der Bediener das Bedienelement 6 um die Längsmittelachse 50. Dabei dreht sich der über die Kupplung 16 drehfest mit dem Bedienelement 6 verbundene Mitnehmer 14 mit. Der Mitnehmer 14 ist über die drehfeste Verbindung 24 mit dem Dosierorgan 18 verbunden, das sich ebenfalls mit dreht. Die Kolbenstange 12 ist über das Verbindungselement 56 drehfest mit dem Bedienelement 6 verbunden und dreht sich ebenfalls mit. Aufgrund der zweiten Gewindeverbindung 19 und der drehfesten Fixierung der Injektionshülse 17 im oberen Gehäuseteil 3 wird die Injektionshülse 17 bei der Drehbewegung des Dosierorgans 18 in distale Richtung 30 bewegt. Dabei bewegt sich die Injektionshülse 17 mit ihrem Anlagerand 27 auf den Anlagerand 25 des Gehäuses 2 zu, wodurch die Feder 9 gespannt wird. Der Anlagerand 25 des Gehäuses 2 kann dabei einen Anschlag für die distale Position der Injektionshülse 17, also auch für die maximal einstellbare Dosis, bilden. Die axiale Position des Bedienelements 6 verändert sich beim Einstellen der auszupressenden Dosis von Injektionsflüssigkeit nicht. Aufgrund der Bewegung der Injektionshülse 17 in distale Richtung verkürzt sich die Länge des Ringraums, in dem die Feder 9 angeordnet ist.

Die Figuren 3 bis 5 zeigen die Gestaltung des Injektionsgeräts 1 im Bereich des Bedienelements 6 und des distalen Endes des oberen Gehäuseteils 3 im Einzelnen. Wie Fig. 3 zeigt, besitzt der Mitnehmer 14 in seinem distalen Bereich einen ersten Abschnitt 45. Das Bedienelement 6 besitzt einen Hülsenabschnitt 49. Der proximale Bereich des Hülsenabschnitts 49 ragt radial zwischen den Abschnitt 45 des Mitnehmers 14 und die Umfangswand des oberen Gehäuseteils 3. Wie Fig. 4 zeigt, weist die Umfangswand des oberen Gehäuseteils 3 an ihrer Innenseite eine Verrastung 41 auf, die durch eine Vielzahl am Umfang gleichmäßig verteilt angeordneter Rastelemente 42 gebildet ist. Am Hülsenabschnitt 49 des Bedienelements 6 sind die Rastarme 36 angeordnet. Wie Fig. 4 zeigt, sind drei Rastarme 36 über den Umfang verteilt angeordnet. Jeder Rastarm 36 trägt an seinem freien Ende ein Rastelement 47, das an dem Rastarm 36 radial nach innen federnd gelagert ist und das mit den Rastelementen 42 zur Festlegung von Raststellungen des Bedienelements 6 zusammenwirkt.

Zum Einstellen einer auszupressenden Menge von Injektionsflüssigkeit wird das Bedienelement 6 in einer ersten Drehrichtung 80 gegenüber dem oberen Gehäuseteil 3 gedreht. Die erste Drehrichtung 80 verläuft bei Blickrichtung in proximale Richtung, also vom Bedienelement 6 in Richtung auf die Injektionsnadel 8, im Uhrzeigersinn. Wie die Fig. 4 und 5 zeigen, sind die Rastelemente 42 unsymmetrisch ausgebildet. Ein Verdrehen des Bedienelements 6 in einer zweiten, der ersten Drehrichtung entgegen gerichteten Drehrichtung 81 wird dadurch von der ersten Rasteinrichtung 35 verhindert. Beim Verdrehen des Bedienelements 6 in der ersten Drehrichtung 80 erzeugt die erste Rasteinrichtung 35 vom Bediener spür- und hörbare Raststellungen. Ein Verstellen des Bedienelements 6 in eine zwischen zwei Raststellungen liegende Position ist aufgrund der Geometrie des Rastelemente 42 und 47 und aufgrund der von der Feder 9 ausgeübten Kraft in der zweiten Drehrichtung 81, die das Bedienelement 6 in die jeweils nächstliegende, einer niedrigeren Menge an Injektionsflüssigkeit zugeordnete Raststellung zurückstellt, nicht möglich.

Wie Fig. 4 auch zeigt, besitzt das Bedienelement 6 eine Reihe von Raststegen 43, die im Ausführungsbeispiel radial nach außen ragen. Im Ausführungsbeispiel sind sechs Raststege 43 vorgesehen, die in einer Schnittdarstellung senkrecht zur Längsmittelachse 50 als schmale, in radialer Richtung zur Längsmittelachse 50 ausgerichtete Arme ausgebildet sind. Die Raststege 43 ragen in der in den Figuren 3 bis 5 gezeigten ersten, distalen Position 51 des Bedienelements 6 in schlitzförmige Aufnahmen 44 im ersten Abschnitt 45 des Mitnehmers 14. Die Raststege 43 bilden mit den Aufnahmen 44 die Kupplung 16, die das Bedienelement 6 drehfest mit dem Mitnehmer 14 verbindet.

Fig. 5 zeigt einen Schnitt durch einen zweiten Abschnitt 60 des Mitnehmers 14. Der zweite Abschnitt 60 ist etwa zylindrisch ausgebildet und wird von einem Stiftabschnitt 48 des Bedienelements 6 durchragt. Der zweite Abschnitt 60 besitzt an seiner Innenwand eine Verrastung 63, die durch eine Vielzahl von am Umfang gleichmäßig verteilt angeordneten Rastelementen 64 gebildet ist. Die Funktion der Verrastung 63 wird im Folgenden noch näher erläutert.

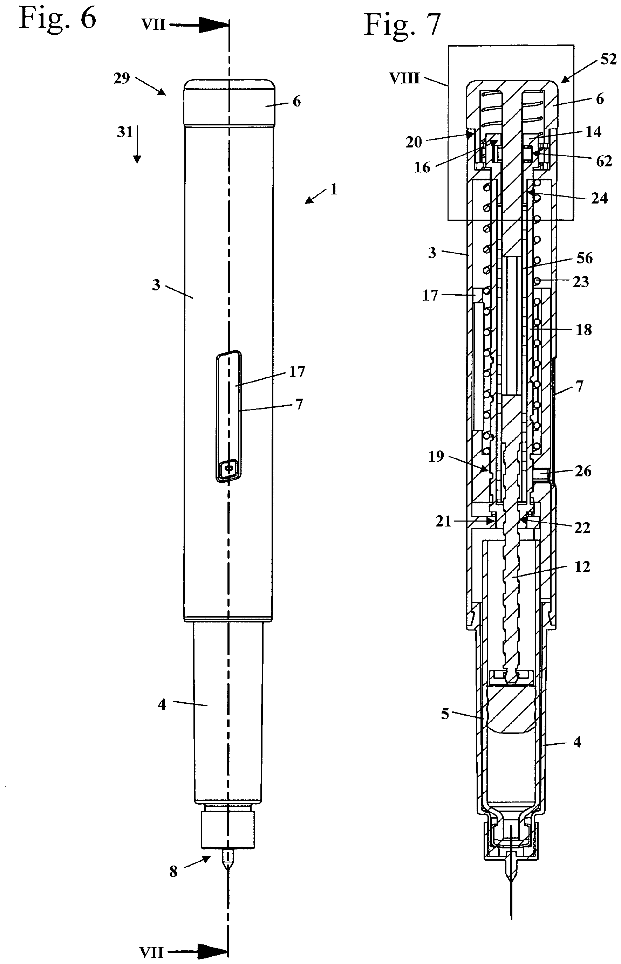

Die Figuren 6 und 7 zeigen das Injektionsgerät 1 in einer Endstellung 29 unmittelbar nach dem Auspressen einer auszupressenden Menge an Injektionsflüssigkeit. Das Bedienelement 6 befindet sich in seiner zweiten, proximalen Position 52. Die zweite Kupplung 20 ist geschlossen und verbindet das Bedienelement 6 drehfest mit dem oberen Gehäuseteil 3, so dass sich das Bedienelement 6 nicht gegenüber dem Gehäuse 2 drehen kann. Die erste Kupplung 16 ist geöffnet, so dass sich der Mitnehmer 14 gegenüber dem Bedienelement 6 drehen kann. Um die Endstellung 29 zu erreichen, hat der Bediener das Bedienelement 6 aus der in den Figuren 1 und 2 gezeigten Nullstellung 28 in proximaler Richtung 31 bewegt, wie in Fig. 6 angedeutet ist. Dadurch sind die Stege 38 der Kupplung 20 in Eingriff mit den Rastelementen 42 der Rasteinrichtung 35 gelangt (Fig. 9). Dadurch wurde das Bedienelement 6 gegenüber dem oberen Gehäuseteil 3 drehfest fixiert. Aufgrund der Bewegung des Bedienelements 6 in proximale Richtung wurden die Raststege 43 aus den Aufnahmen 44 bewegt und dadurch die Kupplung 16 gelöst, so dass sich der Mitnehmer 14 gemeinsam mit dem Dosierorgan 18 um die Längsmittelachse 50 drehen konnte. Die Drehbewegung erfolgte aufgrund der von der gespannten Feder 9 auf die Injektionshülse 17 ausgeübten axialen Kraft, die eine Drehung des Dosierorgans 18 bewirkte. Die Drehung erfolgte aufgrund der Gewindeverbindung 19 und der drehfesten Führung der Injektionshülse 17 im oberen Gehäuseteil 3. Die Kolbenstange 12 ist über das Verbindungselement 56 und das Bedienelement 6 drehfest mit dem oberen Gehäuseteil 3 verbunden. Bei der Drehung des Dosierorgans 18 bewirkt die Gewindeverbindung 22 deshalb eine Bewegung der Kolbenstange 12 in proximale Richtung 31. Dadurch wurde die eingestellte Menge an Injektionsflüssigkeit aus dem Behälter 5 ausgepresst, bis die in den Figuren 6 und 7 gezeigte Endstellung 29 erreicht wurde.

Die Figuren 8 bis 10 zeigen die Kupplung 20 und eine zweite Rasteinrichtung 62 im Einzelnen. Wie Fig. 9 zeigt, sind insgesamt drei Stege 38 vorgesehen, die zwischen Rastelemente 42 des oberen Gehäuseteils 3 ragen und dadurch das Bedienelement 6 drehfest mit dem oberen Gehäuseteil 3 verbinden. Wie Fig. 9 auch zeigt, haben sich die Raststege 43 aus den Aufnahmen 44 im Mitnehmer 14 bewegt, so dass die erste Kupplung 16 geöffnet ist und eine Relativdrehung des Mitnehmers 14 gegenüber dem Bedienelement 6 erlaubt.

Wie Fig. 10 zeigt, sind die Raststege 43 in der Endstellung 29 im Bereich der Verrastung 63 mit den Rastelementen 64 angeordnet und bilden mit diesen die zweite Rasteinrichtung 62. Die Raststege 43 bilden demnach sowohl einen Teil der Kupplung 16 als auch einen Teil der Rasteinrichtung 62. Auch die Rastelemente 64 der Verrastung 63 sind unsymmetrisch ausgebildet und lassen eine Drehung des Mitnehmers 14 gegenüber dem Bedienelement 6 in der zweiten Drehrichtung 81 zu. In der ersten Drehrichtung 80 wird eine Drehung des Mitnehmers 14 gegenüber dem Bedienelement 6 aufgrund der Ausrichtung der Rastflanken etwa radial zur Längsmittelachse 50 verhindert.

Die erste Rasteinrichtung 35 bestimmt die Stärke der beim Einstellen einer auszupressenden Menge von Injektionsflüssigkeit spür- und hörbaren Rastschritte. Beim Einstellen der auszupressenden Menge an Injektionsflüssigkeit ist die zweite Rasteinrichtung 62 nicht wirksam, da die Raststege 43 sich nicht im Bereich der Verrastung 63 befinden. Vielmehr sind das Bedienelement 6 und der Mitnehmer 14 über die Raststege 43 drehfest miteinander verbunden. Zum Auslösen einer Injektion wird das Bedienelement 6 vom Bediener aus der distalen Position 51 in die proximale Position 52 bewegt. Beim Auspressen von Injektionsflüssigkeit befindet sich das Bedienelement 6 dadurch in der zweiten proximalen Position 52. In dieser Position ist die erste Rasteinrichtung 35 nicht wirksam, da die Kupplung 20 das Bedienelement drehfest mit dem oberen Gehäuseteil 3 verbindet. Die Kupplung 16 zwischen Bedienelement 6 und Mitnehmer 14 ist gelöst. Die zweite Rasteinrichtung 62 ist aktiv. Die zweite Rasteinrichtung 62 legt die Stärke der spür- und hörbaren Rastschritte beim Auspressen einer auszupressenden Menge an Injektionsflüssigkeit fest. Dadurch, dass die beiden Rasteinrichtungen 35 und 62 unabhängig voneinander ausgebildet sind und jeweils nur eine der beiden Rasteinrichtungen 35 oder 62 wirksam ist, kann die Stärke der Rastschritte beim Einstellen einer auszupressenden Menge an Injektionsflüssigkeit und beim Auspressen von Injektionsflüssigkeit unabhängig voneinander ausgelegt werden. Auch die Anzahl der Rastschritte kann grundsätzlich unterschiedlich sein.

Nach dem Lösen der Kupplung 16 erfolgt die Injektion aufgrund der in der Feder 9 gespeicherten Kraft automatisch. Die Feder 9 ist dabei so ausgelegt, dass die in der Feder 9 gespeicherte Kraft ausreicht, um den Widerstand des Stopfens 10 und den durch die zweite Rasteinrichtung 62 ausgeübten Widerstand zu überwinden und Injektionsflüssigkeit aus dem Behälter 5 auszupressen.

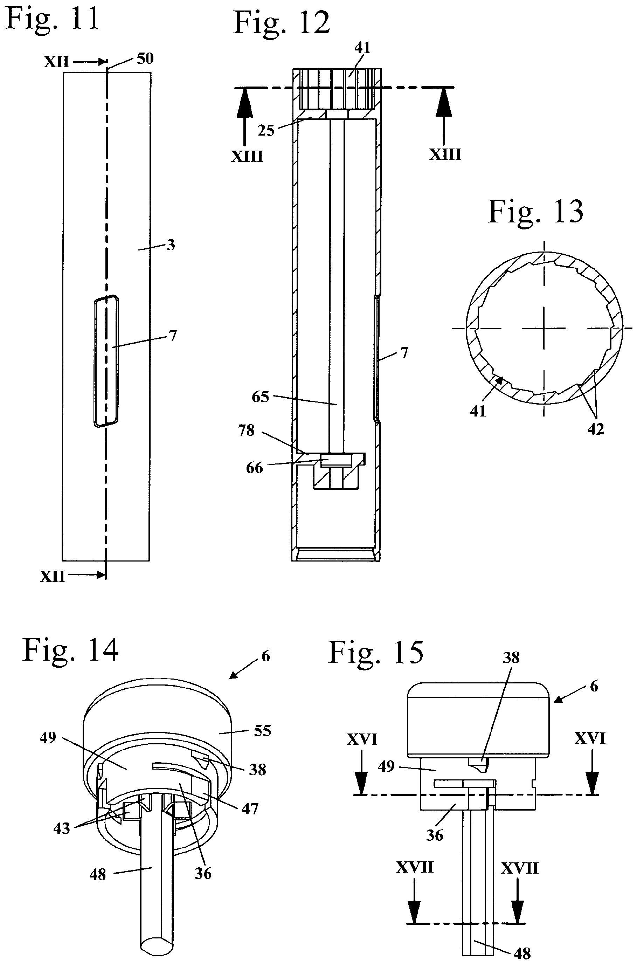

Die Fig. 11 bis 13 zeigen die Gestaltung des oberen Gehäuseteils 3 im Einzelnen. In Fig. 11 ist das Sichtfenster 7 gezeigt, das sich parallel zur Längsmittelachse 50 erstreckt. Wie Fig. 12 zeigt, besitzt das obere Gehäuseteil 3 an seinem distalen Ende am Innenumfang die Verrastung 41, wie auch Fig. 13 zeigt. Die Verrastung 41 erstreckt sich, wie Fig. 12 zeigt, im Ausführungsbeispiel bis zu dem senkrecht zur Längsmittelachse 50 verlaufenden Anlagerand 25, der zur Lagerung des Mitnehmers 14 dient und an dem sich die Feder 9 abstützt. Das obere Gehäuseteil 3 besitzt eine Querwand 78, an der sich das Dosierorgan 18 abstützt. Die Querwand 78 besitzt eine Lageröffnung 66, in der das Dosierorgan 18 drehbar gelagert ist. An der Innenseite des oberen Gehäuseteils 3 sind Führungsstege 65 vorgesehen, in denen die Injektionshülse 17 axial verschiebbar und drehfest gegenüber dem oberen Gehäuseteil 3 geführt ist.

Die Figuren 14 bis 17 zeigen die Gestaltung des Bedienelements 6 im Einzelnen. Das Bedienelement 6 besitzt einen Bedienabschnitt 55, der aus dem Gehäuse 2 ragt und an dem der Bediener das Bedienelement 6 gegenüber dem oberen Gehäuseteil 3 beim Einstellen einer auszupressenden Menge an Injektionsflüssigkeit drehen kann. An der proximalen Seite des Bedienabschnitts 55 ist der Hülsenabschnitt 49 angeordnet, der an seiner Außenseite die Stege 38 der Kupplung 20 sowie die Rastarme 36 der ersten Rasteinrichtung 35 trägt. Der Hülsenabschnitt 49 ist von dem Stiftabschnitt 48 durchragt. Innerhalb des Hülsenabschnitts 49 sind am Stiftabschnitt 48 die Raststege 43 angeordnet.

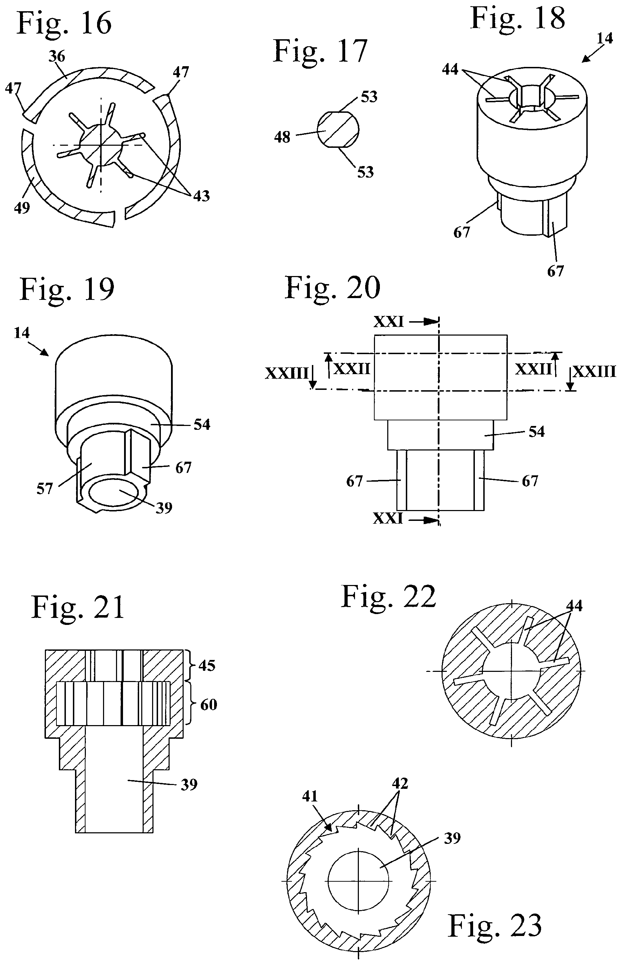

Fig. 16 zeigt die Anordnung der Raststege 43 innerhalb des Hülsenabschnitts 49. Wie Fig. 17 zeigt, besitzt der Stiftabschnitt 48 an seiner Außenseite zwei einander gegenüber liegende Abflachungen 53, die zur drehfesten Verbindung mit dem Verbindungselement 56 (Fig. 2) dienen.

Die Figuren 18 bis 23 zeigen den Mitnehmer 14 im Einzelnen. Der Mitnehmer 14 besitzt einen Lagerabschnitt 54, mit dem der Mitnehmer 14 am Anlagerand 25 drehbar gelagert ist. An der proximalen Seite des Lagerabschnitts 54 besitzt der Mitnehmer 14 einen Hülsenabschnitt 57, an dessen Außenseite parallel zur Längsmittelachse 50 verlaufende Sicherungsstege 67 vorgesehen sind. Die Sicherungsstege 67 dienen zur drehfesten Verbindung des Mitnehmers 14 mit dem Dosierorgan 18. Der Mitnehmer 14 besitzt eine durchgehende Öffnung 39, durch die der Stiftabschnitt 48 des Bedienelements 6 ragt, wie Fig. 8 zeigt.

Wie die Figuren 21 bis 23 zeigen, besitzt der Mitnehmer 14 den ersten Abschnitt 45, in dem die Aufnahmen 44 der Kupplung 16 ausgebildet sind, sowie den zweiten Abschnitt 60, in dem die Verrastung 41 mit den Rastelementen 42 angeordnet ist. Der zweite Abschnitt 60 ist dabei an der proximalen Seite des ersten Abschnitts 45 angeordnet.

Die Figuren 24 bis 26 zeigen das Dosierorgan 18 im Einzelnen. Das Dosierorgan 18 besitzt an seiner proximalen Seite einen Lagerstutzen 72, mit dem das Dosierorgan 18 in der Lageröffnung 66 des oberen Gehäuseteils 3 gelagert ist. Benachbart zum Lagerstutzen 72 sind Anlagestege 73 vorgesehen, die stirnseitig an der Querwand 78 des oberen Gehäuseteils 3 anliegen und die die Reibung zwischen dem Dosierorgan 18 und dem oberen Gehäuseteil 3 verringern. Am Außenumfang des Dosierorgans 18 ist die Skala 71 aufgebracht. Das Dosierorgan 18 trägt im Bereich der Skala 71 ein Außengewinde 70, das mit einem in Fig. 28 gezeigten Innengewinde 77 der Injektionshülse 17 zusammenwirkt und mit diesem die Gewindeverbindung 19 bildet.

Wie Fig. 25 zeigt, besitzt das Dosierorgan 18 an seinem distalen Ende zwei Vertiefungen 69, in die die Sicherungsstege 67 des Mitnehmers 14 (Fig. 19) zur drehfesten Verbindung des Mitnehmers 14 mit dem Dosierorgan 18 ragen. Wie Fig. 26 zeigt, besitzt das Dosierorgan 18 am Lagerstutzen 72 ein Innengewinde 74, das mit dem Außengewinde 46 der Kolbenstange 12 zusammenwirkt und mit diesem die Gewindeverbindung 22 bildet.

Die Figuren 27 bis 29 zeigen die Injektionshülse 17 im Einzelnen. Die Injektionshülse 17 besitzt in Richtung der Längsmittelachse 50 verlaufende Aussparungen 75, die zur Gewichtsreduzierung dienen. Wie Fig. 28 zeigt, besitzt die Injektionshülse 17 einen Steg 34, der in proximale Richtung ragt. Der Steg 34 überdeckt in der distalen Position der Injektionshülse 17 den proximalen Teil der Skala 71. Wie Fig. 28 auch zeigt, ist die Öffnung 26 im Bereich des Innengewindes 77 angeordnet.

Wie Fig. 29 zeigt, besitzt die Injektionshülse 17 an ihrem Außenumfang Führungsnuten 76, die zur drehfesten Verbindung mit dem oberen Gehäuseteil 3 dienen und in die die Führungsstege 65 des oberen Gehäuseteils 3 ragen.

Die vorliegende Erfindung kann auch bei einem manuellen Injektionsgerät, also bei einem Injektionsgerät, bei dem die Injektionsflüssigkeit vom Bediener durch Drücken auf das Bedienelement 6 ausgepresst wird, vorteilhaft sein. Auch bei einem solchen Injektionsgerät können ein Bedienelement 6 und ein Mitnehmer 14 vorgesehen sein, die sich beim Einstellen einer auszupressenden Menge an Injektionsflüssigkeit in einer ersten axialen Relativposition zueinander befinden und beim Auspressen der Injektionsflüssigkeit in einer zweiten axialen Position zueinander. In einer bevorzugten Gestaltung umfasst die zweite Rasteinrichtung eine an der Stirnseite des Mitnehmers angeordnete Stirnverzahnung, die mit in Richtung der Längsmittelachse 50 auf den Mitnehmer zu ragenden Armen des Bedienelements 6 zusammenwirkt. Eine solche Rasteinrichtung ist in Abhängigkeit der relativen Lage von Bedienelement und Mitnehmer zueinander wirksam oder nicht wirksam. Es ist vorteilhaft vorgesehen, dass die Rasteinrichtung in der ersten, distalen Position des Bedienelements nicht in Eingriff mit der Stirnverzahnung ist und dadurch nicht wirksam ist. In der zweiten, proximalen Position des Bedienelements ist der mindestens eine Arm vorteilhaft in Eingriff mit der Stirnverzahnung, so dass die Rasteinrichtung in dieser Relativposition wirksam ist. Die Rasteinrichtung ist dabei vorteilhaft so ausgelegt, dass eine Drehung des Mitnehmers gegenüber dem Bedienelement nur in einer Richtung möglich ist. Die Rasteinrichtung besitzt damit die Funktion eines Freilaufs.

Auch andere Gestaltungen der Rasteinrichtungen können vorteilhaft sein.

## Patentansprüche

1. Injektionsgerät mit einem Gehäuse (2), mit einer Längsmittelachse (50), mit einem Bedienelement (6) und mit einem drehbar und in Richtung der Längsmittelachse (50) fest im Gehäuse (2) gehaltenen Dosierorgan (18), wobei das Bedienelement (6) in einer ersten Position (51) zur Einstellung einer auszupressenden Menge an Injektionsflüssigkeit gegenüber dem Gehäuse (2) drehbar gelagert ist und über eine erste Kupplung (16) drehfest mit dem Dosierorgan (18) verbunden ist, wobei das Bedienelement (6) in einer zweiten Position (52) zum Auspressen einer auszupressenden Menge an Injektionsflüssigkeit über eine zweite Kupplung (20) drehfest mit dem Gehäuse (2) verbunden ist und gegenüber dem Dosierorgan (18) drehbar ist, und wobei zwischen dem Bedienelement (6) und dem Gehäuse (2) eine erste Rasteinrichtung (35) wirkt, die nur in der ersten Position (51) des Bedienelements (6) beim Einstellen einer auszupressenden Menge an Injektionsflüssigkeit wirksam ist,
**dadurch gekennzeichnet, dass** zwischen dem Bedienelement (6) und dem Dosierorgan (18) eine zweite Rasteinrichtung (62) wirkt, die nur in der zweiten Position (52) des Bedienelements (6) beim Auspressen einer auszupressenden Menge an Injektionsflüssigkeit wirksam ist.

2. Injektionsgerät nach Anspruch 1,
**dadurch gekennzeichnet, dass** das Dosierorgan (18) sich beim Einstellen einer auszupressenden Menge an Injektionsflüssigkeit in einer ersten Drehrichtung (80) gegenüber dem Gehäuse (2) dreht und beim Auspressen einer eingestellten Menge an Injektionsflüssigkeit in einer der ersten Drehrichtung (80) entgegen gerichteten zweiten Drehrichtung (81) gegenüber dem Gehäuse (2) dreht.

3. Injektionsgerät nach Anspruch 2,
**dadurch gekennzeichnet, dass** die zweite Rasteinrichtung (62) eine Drehung des Dosierorgans (18) gegenüber dem Bedienelement (6) in der zweiten Drehrichtung (81) erlaubt und eine Drehung des Dosierorgans (18) gegenüber dem Bedienelement (6) in der ersten Drehrichtung (80) sperrt.

4. Injektionsgerät nach einem der Ansprüche 1 bis 3,
**dadurch gekennzeichnet, dass** das Injektionsgerät (1) eine Feder (23) besitzt, die beim Einstellen einer auszupressenden Menge von Injektionsflüssigkeit gespannt wird und die bei gelöster erster Kupplung (16) das Auspressen von Injektionsflüssigkeit bewirkt.

5. Injektionsgerät nach einem der Ansprüche 1 bis 4,
**dadurch gekennzeichnet, dass** das Bedienelement (6) mindestens einen Raststeg (43) besitzt, wobei der mindestens eine Raststeg (43) in der ersten Position (51) des Bedienelements (6) einen Teil der ersten Kupplung (16) bildet und in der zweiten Position (52) des Bedienelements (6) einen Teil der zweiten Rasteinrichtung (62) bildet.

6. Injektionsgerät nach einem der Ansprüche 1 bis 5,
**dadurch gekennzeichnet, dass** das Injektionsgerät (1) einen Mitnehmer (14) besitzt, der drehfest mit dem Dosierorgan (18) verbunden ist.

7. Injektionsgerät nach Anspruch 6,
**dadurch gekennzeichnet, dass** die zweite Rasteinrichtung (62) an dem Bedienelement (6) und dem Mitnehmer (14) ausgebildet ist.

8. Injektionsgerät nach Anspruch 6 oder 7,
**dadurch gekennzeichnet, dass** die erste Kupplung (16) an dem Bedienelement (6) und dem Mitnehmer (14) ausgebildet ist.

9. Injektionsgerät nach einem der Ansprüche 1 bis 8,
**dadurch gekennzeichnet, dass** das Injektionsgerät (1) einen Dosierkolben (11) besitzt, der drehfest mit dem Bedienelement (6) verbunden ist und der über eine erste Gewindeverbindung (22) mit dem Dosierorgan (18) verbunden ist.

10. Injektionsgerät nach einem der Ansprüche 1 bis 9,
**dadurch gekennzeichnet, dass** das Injektionsgerät (1) eine Injektionshülse (17) besitzt, die drehfest und in Richtung der Längsmittelachse (50) verschiebbar gegenüber dem Gehäuse (2) gehalten ist und die über eine zweite Gewindeverbindung (19) mit dem Dosierorgan (18) verbunden ist.

## Claims

1. Injection device having a housing (2), a central longitudinal axis (50), an operating element (6) and a dosing member (18) held rotatably in the housing (2) while being fixed in the direction of the central longitudinal axis (50), wherein the operating element (6) is in a first position (51) mounted rotatably with respect to the housing (2) and non-rotatably connected to the dosing member (18) by way of a first coupling element (16) for setting a quantity of injection fluid to be expressed, wherein the operating element (6) is in a second position (52) non-rotatably connected to the housing (2) by way of a second coupling element (20) and rotatable with respect to the dosing member (18) for expressing a quantity of injection fluid to be expressed, and wherein a latching device (35), which is only operative in the first position (51) of the operating element (6) when setting a quantity of injection fluid to be expressed, acts between the operating element (6) and the housing (2), **characterised in that** a second latching device (62), which is only operative in the second position (52) of the operating element (6) when expressing a quantity of injection fluid to be expressed, acts between the operating element (6) and the dosing member (18).

2. Injection device according to claim 1,
**characterised in that** the dosing member (18) rotates in a first direction of rotation (80) with respect to the housing (2) when setting a quantity of injection fluid to be expressed and in a second direction of rotation (81) opposed to the first direction of rotation (80) with respect to the housing (2) when expressing a set quantity of injection fluid.

3. Injection device according to claim 2,
**characterised in that** the second latching device (62) allows a rotation of the dosing member (18) with respect to the operating element (6) in the second direction of rotation (81) and blocks a rotation of the dosing member (18) with respect to the operating element (6) in the first direction of rotation (80).

4. Injection device according to any of claims 1 to 3,
**characterised in that** the injection device (1) has a spring (23), which is tensioned when setting a quantity of injection fluid to be expressed and which causes the expression of injection fluid when the first coupling element (16) is released.

5. Injection device according to any of claims 1 to 4,
**characterised in that** the operating element (6) has at least one latching web (43), wherein the least one latching web (43) forms a part of first coupling element (16) in the first position (51) of the operating element (6) and forms a part of the second latching device (62) in the second position (52) of the operating element (6).

6. Injection device according to any of claims 1 to 5,
**characterised in that** the injection device (1) has an entrainer (14), which is non-rotatably connected to the dosing member (18).

7. Injection device according to claim 6,
**characterised in that** the second latching device (62) is formed at the operating element (6) and the entrainer (14).

8. Injection device according to claim 6 or 7,
**characterised in that** the first coupling element (16) is formed at the operating element (6) and the entrainer (14).

9. Injection device according to any of claims 1 to 8,
**characterised in that** the injection device (1) has a metering piston (11), which is non-rotatably connected to the operating element (6) and connected to the dosing member (18) via a first threaded connection (22).

10. Injection device according to any of claims 1 to 9,
**characterised in that** the injection device (1) has an injection sleeve (17), which is held non-rotatably and displaceable in the direction of the central longitudinal axis (50) with respect to the housing (2) and which is connected to the dosing member (18) via a a second threaded connection (19).

## Revendications

1. Appareil d'injection avec un boîtier (2), avec un axe longitudinal médian (50), avec un élément de manœuvre (6) et avec un organe doseur (18) retenu à rotation et de manière fixe dans la direction de l'axe longitudinal médian (50) dans le boîtier (2), dans lequel l'élément de manœuvre (6) est monté à rotation par rapport au boîtier (2) dans une première position (51) pour régler une quantité de liquide d'injection à expulser et est relié fixe en rotation à l'organe doseur (18) via un premier accouplement (16), dans lequel l'élément de manœuvre (6) est relié fixe en rotation au boîtier (2) via un deuxième accouplement (20) dans une deuxième position (52) pour expulser une quantité de liquide d'injection à expulser et est apte à tourner par rapport à l'organe doseur (18), et dans lequel entre l'élément de manœuvre (6) et le boîtier (2) agit un premier dispositif d'encliquetage (35) qui n'est actif que dans la première position (51) de l'élément de manœuvre (6) lors du réglage d'une quantité de liquide d'injection à expulser,
**caractérisé en ce qu'**entre l'élément de manœuvre (6) et l'organe doseur (18) agit un deuxième dispositif d'encliquetage (62) qui n'est actif que dans la deuxième position (52) de l'élément de manœuvre (6) lors de l'expulsion d'une quantité de liquide d'injection à expulser.

2. Appareil d'injection selon la revendication 1,
**caractérisé en ce que** l'organe doseur (18), lors du réglage d'une quantité de liquide d'injection à expulser, tourne dans une première direction de rotation (80) par rapport au boîtier (2) et, lors de l'expulsion d'une quantité réglée de liquide d'injection, tourne dans une deuxième direction de rotation (81), opposée à la première direction de rotation (80), par rapport au boîtier (2).

3. Appareil d'injection selon la revendication 2,
**caractérisé en ce que** le deuxième dispositif d'encliquetage (62) permet une rotation de l'organe doseur (18) par rapport à l'élément de manœuvre (6) dans la deuxième direction de rotation (81), et bloque une rotation de l'organe doseur (18) par rapport à l'élément de manœuvre (6) dans la première direction de rotation (80).

4. Appareil d'injection selon l'une des revendications 1 à 3,
**caractérisé en ce que** l'appareil d'injection (1) a un ressort (23) qui, lors du réglage d'une quantité de liquide d'injection à expulser, est en tension et qui, quand le premier accouplement (16) est débloqué, provoque l'expulsion de liquide d'injection.

5. Appareil d'injection selon l'une des revendications 1 à 4,
**caractérisé en ce que** l'élément de manœuvre (6) a au moins une ailette d'encliquetage (43), dans lequel la au moins une ailette d'encliquetage (43), dans la première position (51) de l'élément de manœuvre (6), forme une partie du premier accouplement (16) et, dans la deuxième position (52) de l'élément de manœuvre (6), forme une partie du deuxième dispositif d'encliquetage (62).

6. Appareil d'injection selon l'une des revendications 1 à 5,
**caractérisé en ce que** l'appareil d'injection (1) a un organe d'entraînement (14) qui est relié fixe en rotation à l'organe doseur (18).

7. Appareil d'injection selon la revendication 6,
**caractérisé en ce que** le deuxième dispositif d'encliquetage (62) est formé sur l'élément de manœuvre (6) et sur l'organe d'entraînement (14).

8. Appareil d'injection selon la revendication 6 ou 7,
**caractérisé en ce que** le premier accouplement (16) est formé sur l'élément de manœuvre (6) et sur l'organe d'entraînement (14).

9. Appareil d'injection selon l'une des revendications 1 à 8,
**caractérisé en ce que** l'appareil d'injection (1) a un piston doseur (11) qui est relié fixe en rotation à l'élément de manœuvre (6) et qui est relié par une première liaison à filetage (22) à l'organe doseur (18).

10. Appareil d'injection selon l'une des revendications 1 à 9,
**caractérisé en ce que** l'appareil d'injection (1) a un manchon d'injection (17) qui est retenu par rapport au boîtier (2) en étant fixe en rotation et coulissant dans la direction de l'axe longitudinal médian (50) et qui est relié par une deuxième liaison à filetage (19) à l'organe doseur (18).
